# EUROPEAN PATENT SPECIFICATION

(11) **EP 2 806 839 B1**
(45) Date of publication and mention of the grant of the patent: **07.07.2021**
(21) Application number: 13712332.9
(22) Date of filing: 28.01.2013
(51) Int. Cl.: A61F 9/00

(54) **DEVICE FOR THE TRANSPLANTATION OF CELLS IN SUSPENSION**
VORRICHTUNG ZUR TRANSPLANTATION VON ZELLEN IN EINER SUSPENSION
DISPOSITIF DE TRANSPLANTATION DE CELLULES EN SUSPENSION

(30) Priority: 27.01.2012 EP 12152866
(43) Date of publication of application: 03.12.2014
(73) Proprietor: École Polytechnique Fédérale de Lausanne (EPFL), 1015 Lausanne (CH)
(72) Inventor: BARRANDON, Yann, 1026 Echandens-Denges (CH); VAN DEN BERGH, Hubert, CH-1376 Goumoens-la-Ville (CH); RENAUD, Philippe, CH-1028 Préverenges (CH); BRASCHLER, Thomas, 1022 Chavannes-Renens VD (CH); GRABER, Julien, CH-1008 Prilly (CH); CAILLIER, Maïa, CH-1700 Fribourg (CH)
(74) Representative: Grosfillier, Philippe
(86) International application number: PCT/IB2013/050721
(87) International publication number: WO 2013/111121

(56) References cited:
- WO-A1-03/020172
- CN-Y- 2 560 370
- US-A- 3 867 519
- US-A- 4 186 184
- US-A1- 2006 034 891
- US-A1- 2007 106 394

## Description

### OBJECT OF THE INVENTION

The present invention relates to a support containing cavities (or reservoirs), preferably micro-cavities or micro-reservoirs, in direct contact with tissue (e.g., human or animal tissues) to which the support according to the invention is applied allowing direct transplantation of cells on said tissues.

More specifically, in one embodiment, the present invention relates to a contact lens which comprises at least one cavity forming a reservoir containing cells in suspension, said cavity or said cavities being in direct contact with the eye which allows direct transplantation of cells on the eye in a localized manner.

The number, the shape and the volume of the cavities may vary. Similarly one may consider different shapes of support so that it may be adapted to different types of tissues. This support can be achieved in different types of materials, and may be treated on its inner surface in contact with the tissues, e.g. coated with an adhesive substance like a surgical glue or another product according to needs.

### STATE OF THE ART

Publication WO 2008/144340 describes a scleral lens for drug delivery. This scleral lens comprises a reservoir on the whole surface of the cornea, said reservoir containing a drug. This lens does not allow the delivery of the drug in a localized manner but only on the whole of the cornea.

Publication WO 2010/068281 describes a lens for delivering an active agent to the cornea of the eye of a patient. In this document, the lens comprises a carrier material to release the agent which is encapsulated within the lens.

Publication US 2009/269391 describes a contact lens comprising fibers that are used as a means to deliver drugs. The fibers are loaded with drugs and deliver them to the eye.

Publication WO 2009/03226 describes a system for administering an agent ophthalmically bioactive including a contact lens with microparticles or microgels of a crosslinked polymer dispersed therein, said microparticles or microgels having trapped a bioactive agent capable of migration by diffusion in the lacrymal film.

Publication US 2008/075757 describes a lens having a micro-emulsion of oil in water, with a bioactive agent which is ophthalmically encapsulated in the oil phase.

Publication WO 2006/084275 describes a system for delivering drugs using a polymeric hydrogel.

Publication DE 40 22 553 describes a product for the issuance of a substance on the cornea of a patient.

Publication DE 1964116 describes a lens with a reservoir in the shape of ridge which extends inside the lens along its periphery. Such a configuration does not allow targeted application of the product contained in the reservoir.

Publication US 2008/0107713 describes a contact lens with application areas of drugs distributed asymmetrically on the lens. More particularly, the lens includes a first lens made in a non-hydrophilic material and which contains the drug and a second lens on the concave surface of the first lens so as to take the drug in "sandwich", the second lens being made of a hydrophilic material.

Document CN-Y-2560370 discloses a rheumatism plaster for administering medical liquids.

Other publications of the state of the art are: US 2008/317819, US 2008/107713, WO 2007/002671, US 2006/290882, US 2005/196428, WO 03/090662, US 2003/203001, EP0882996, JP 6273702, DE 4022553, US 3786812, CN 2914127Y, EP 0347043, DE 1964116, WO 2008/131973, WO 2007/092550, WO 84/01297, US 4,466,705, US 2008/212021, US 2006/152674, WO 2004 / 046768, US 7695135, WO 93/07840, WO 2009/145842, WO 99/45869, WO 2010/105130, WO 2008/098299, US 3867519, US 2010/069857, WO 2006/029332, US 4186184, US 3961628, WO 03/077794, US 4177256, US 2010/210996, WO 00/66139, WO 95/01764, US 2005/275799, US 2005/013806, US 2005/177149, WO 2006/047788, JP 2002331025, WO 95/20969 , US 2004/081643, WO 2010/33853, WO 03/030959, US 2005/070942, WO 01/30375, WO 03/077898, WO 98/53806, WO 03/030958, WO 02/06883, US 6,541,028, US 2004/049268, US 5932205, WO 2004/060348, WO 2011/031852, US 2007/106394, WO 2005/123035, WO 2006/102378.

### PRINCIPLE OF THE INVENTION

An object of the invention is to improve the known products.

Another object of the invention is to provide a support such as a lens or another support that can be used in a simple and precise way to treat a patient, namely for the application of cells directly on tissue of the patient, typically on the patient's eye as in one of the embodiments of the invention.

The present invention is innovative especially in that it enables the application of a cell suspension (cells which are thus directly transplanted onto the target organ) and that said application has a precise location (determined by the arrangement of cavities of the device).

The device for the application of a medium in the form of a cell suspension, comprises a carrier with at least one micro-cavity for containing said medium in direct contact with the object on which the said medium is to be applied.

The carrier may comprise several micro-cavities.

According to the present invention, the carrier comprises at least one channel for the supply of the micro-cavity or micro-cavities with medium.

According to the present invention, the device further comprises at least one reservoir which allows continuous feeding of the micro-cavity or micro-cavities with medium over time.

In an embodiment the carrier may have the shape of a contact lens.

In an embodiment the carrier may comprise a plurality of cavities that are evenly distributed on the carrier or which are not evenly distributed on the carrier.

In an embodiment, the carrier may comprise protection means to protect the medium from the environment when the carrier is applied to a patient.

In an embodiment the protection means may be a ring or a plurality of cavities.

In an embodiment the cavity or the cavities may have a cylindrical shape, and/or a ring shape and/or have different volumes.

In an embodiment the device may comprise a sensor.

In an embodiment the sensor may be placed in the carrier.

In an embodiment, the sensor may be an indicator dye in the medium or microelectrodes.

In an embodiment the sensor may comprise a polymer or hydrogel pouch.

In an embodiment, in order to reconstitute the medium, the carrier may comprise at least an inlet connected to a medium concentrate chamber covered by a water permeable membrane.

In an embodiment, the inlet may further comprise a valve to build an overpressure in the chamber.

According to the present invention, the at least one cavity contains as a medium cells in suspension for the transplantation of said cells.

In an embodiment, the device may comprise a plurality of cavities, wherein some cavities contain cells in suspension as a medium, and other cavities contain a growth inhibitor, and/or a growth factor, and/or inhibitors of blood vessels, and/or cell death inhibitors and/or a permeabilization agent.

In an embodiment, the sensor may be a pH, CO2 or metabolite concentration sensor.

In an embodiment, the carrier may be adapted for application to human tissue or human or animal body, such as the eye, the gingiva, skin, a wound or a burn or an internal organ.

### DETAILED DESCRIPTION OF THE INVENTION

The present invention will be better understood by the following detailed description and the accompanying figures. Figures 1 to 7 and 10 to 23 do not show embodiments of the invention.
Figures 1 and 2 illustrate the principle of the invention for a lens;
Figure 3 illustrates the application of a lens on the eye of a patient;
Figures 4-7 illustrate a lens;
Figure 8 illustrates an embodiment of the invention;
Figure 9 illustrates another embodiment of the invention;
Figures 10A, 10B and 11 illustrate example;
Figures 12A and 12B illustrate another example;
Figures 13A and 13B illustrate another example;
Figures 14A and 14B illustrate another example;
Figures 15A to 15C illustrate another example;
Figures 16A and 16B illustrate another example;
Figures 17A to 17C illustrate another example;
Figures 18A and 18B illustrate another example;
Figure 19 illustrates another example;
Figures 20A to 20D illustrate another example;
Figures 21A and 21B illustrate another example;
Figures 22A to 22C illustrate another Rule 42 EPC and
Figure 23 illustrates another example.

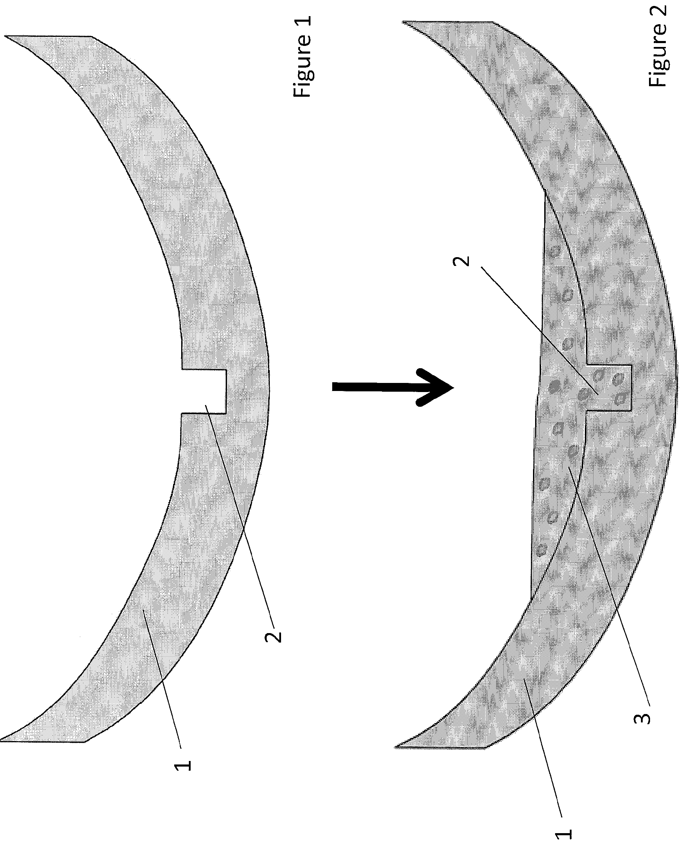

The principle of the present invention is illustrated in Figures 1, 2 and 3. In these figures, one has illustrated a carrier in the shape of a contact lens 1 which comprises at least one microcavity 2. In this microcavity 2, a medium 3 for example a cell suspension is introduced and is intended to come into direct contact with the cornea of a patient (in the case of a contact lens). Because of the small size of the microcavity 2, the medium 3 is maintained within the cavity 2 by means of surface tension forces, which allows the device to easily contact with the tissue or organ to be treated (eye or other)

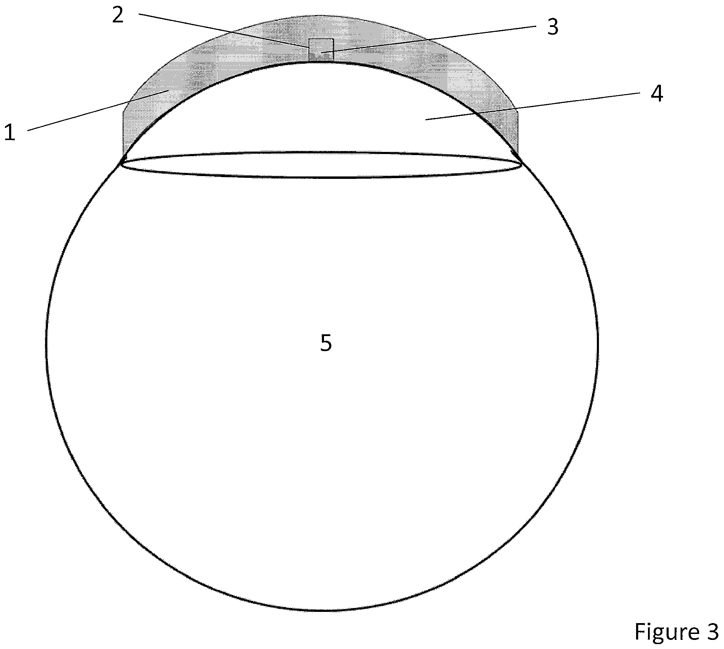

The result of the application of the lens 1 on the cornea of the eye 4 of a user 5 is illustrated in Figure 3.

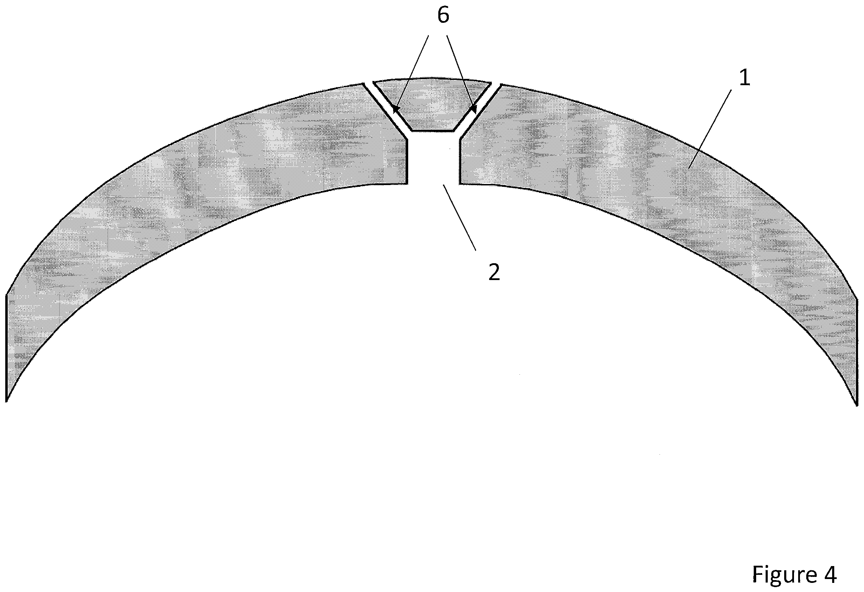

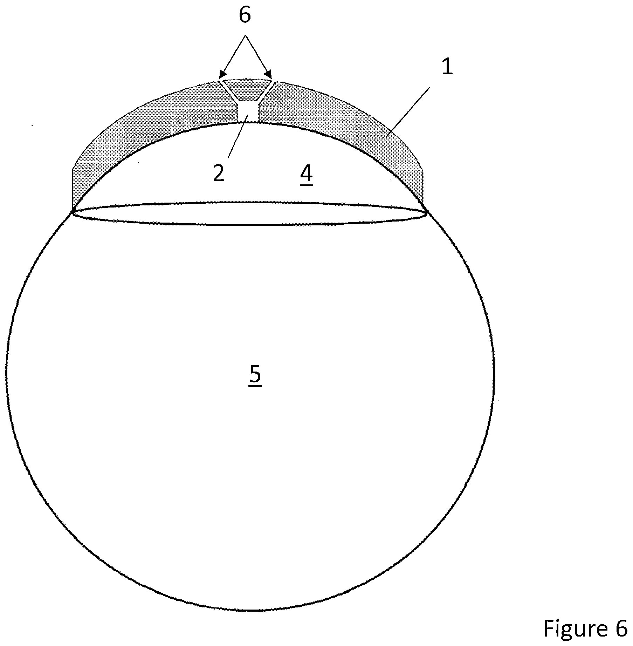

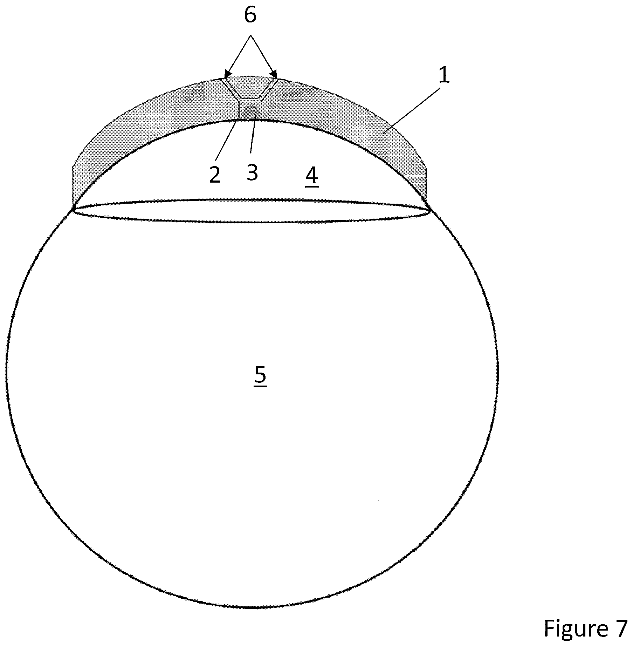

In Figures 4 to 7, an embodiment is shown in which one sees several channels 6 (in fact micro-channels 6) which provide access to the cavity 2 when the carrier (for example a lens 1) is worn by a user (see as illustrated in Figures 3. 6 and 7). It is thus possible to refill the cavity 2, that is to say, to add medium 3 in a cavity without removing or moving the lens (or another support media) for this purpose, see figures 6 and 7. One may add as a medium 3 either the same cell suspension or any other product according to needs and requirements as will be understood from the following description of further embodiments of the present invention.

The channels 6 may comprise a chamfer to facilitate refilling or not and may have any suitable shape (straight or not).

These channels 6 form a particularly interesting feature in that they may hold the carrier 1 (for example a lens) in position on the eye 5. By applying the principle of the invention, it is possible therefore to obtain a support/carrier 1 such as a lens or another equivalent carrier to apply in a very precise and localized manner a cell suspension on a patient. Once the support 1 is positioned appropriately with the cavity 2 placed at the desired location, the support 1 remains in place and the cavity 2 can be fed as long as necessary, without displacing the carrier 1, see figure 7.

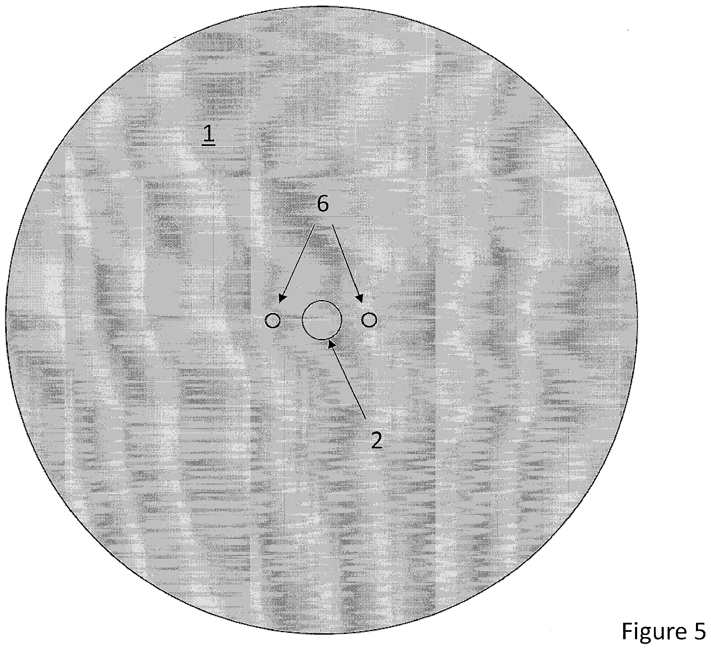

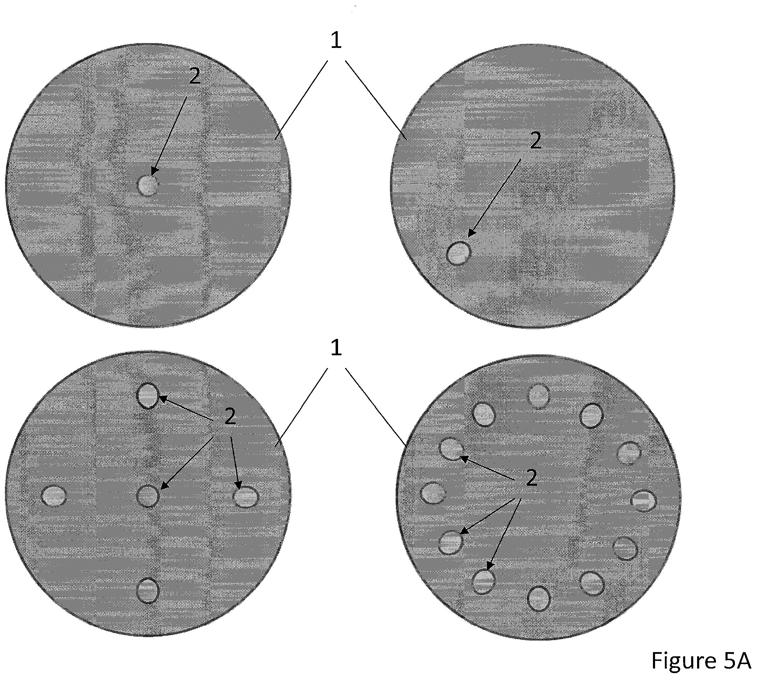

Figure 5 shows a top view of the embodiment of Figure 4 with the same reference numbers identifying the same elements. It should be noted that the number of channels 6 is variable and that they are not necessarily positioned in the center of the device / support 1. The inlets of the channels 6 are preferably placed close to the cavity 2, as shown in Figure 4 for example. Alternatively, depending on the position of the cavity 2, the inlets of the channels 6 may be away from the cavity, so to allow easier access to said inlets. Any other configuration and shape may of course be considered depending on the circumstances in accordance with the principles of the present invention.

Figure 5A illustrates patterns in four different configurations of the cavity (cavities) 2. As it can be understood, the support or carrier 1 (e.g. a lens) may comprise a single cavity 2 centered or not (see the top two illustrations of Figure 5A) two or more cavities evenly distributed or not on the carrier 1 (see the two illustrations at the bottom of Figure 5A).

One of the advantages of the present invention is clear here. One can provide one or more cavities 2 in specific areas of the carrier 1 for a targeted application of cell suspension. In addition, various cavities 2 can contain different products for a complementary application, or mixed substances, once it has reached the target, that is to say the desired location.

By using the channels 6 as illustrated in figures 4, 5-8 in the embodiments of figure 5A, it is also possible to supply/refill all cavities 2 simultaneously or then in turn, either with the same medium 3, or with different products, as required.

In some embodiments all cavities 2 may have at least one channel 6 for filling or refilling with a product, in other embodiments only one or some cavities may have at least one such channel. It is therefore possible to design different embodiments where some selected cavities may be refilled and others not on the same carrier depending on the product used. Some cavities may even be filled optionally, for example after the carrier has been placed on a patient depending on the evolution (to increase the concentration of a product or for any suitable subsequent purpose).

The shape of the cavities 2 can be selected at will, for example circular or oblong, "banana" or other. One may also choose a shape related to the application of the carrier 1 or to the zone where the carrier 1 is to be applied on a patient.

Figure 6 shows the lens 1 of Figure 4 placed on the cornea 4 of an eye 5 of a patient. As shown, in this mode, the micro-cavity 2 is empty, which allows, for example, first to properly position the lens 1 in position before a medium 3 for example a cell suspension is added into the cavity 2 according to the principles of the invention. In the absence of a channel 6, it is of course necessary to add the medium 3 in the cavity before the lens 1 is applied to the eye 5. Of course, the presence of channels 6 does not imply that the lens 1 is necessarily applied without medium 3.

Figure 7 illustrates the system of Figure 4 in which the medium 3 has been introduced into the cavity 2.

As said above, the carrier 1 of figures 6-7 may comprise a plurality of cavities 2 as illustrated in figure 5A.

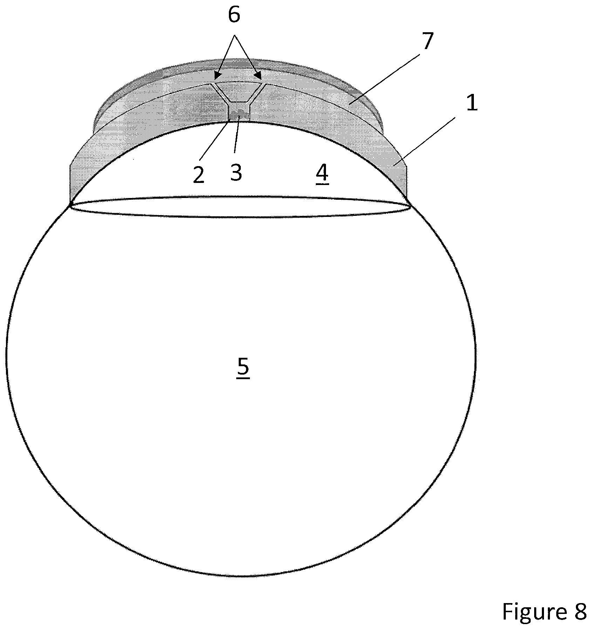

In Figure 8, there is illustrated an embodiment based on that of Figure 7, but in this embodiment of Figure 8, a reservoir 7 has been added over the lens. The reservoir 7 has a capacity normally greater than that of a microcavity 2, sometimes much higher, and allows, through channels (for example channels 6), to supply for a longer period the microcavity 2 with a medium 3, for example a suspension or other type of suspension as initially applied or any other desired product. The carrier may therefore be left in place without being touched for a longer time period.

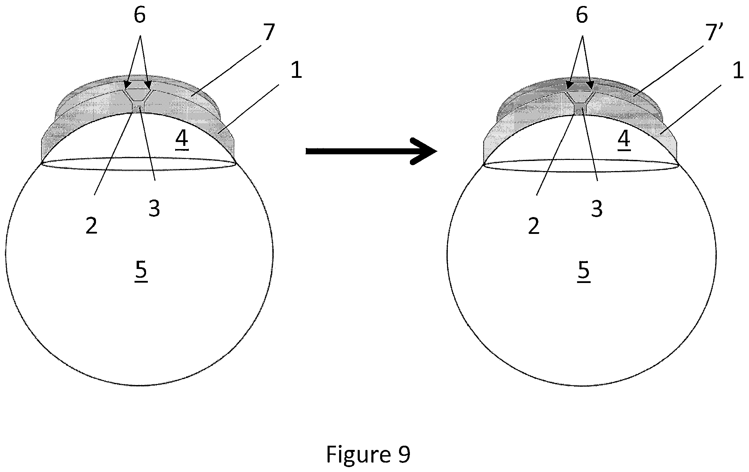

In Figure 9, there is illustrated the principle of change of reservoir 7. As can be understood from this figure, it is possible to change the reservoir 7 for another reservoir 7 without changing or moving the support 1 (lens 1) to renew the medium 3 or replace it with another product, for example growth factors or small molecules promoting proliferation, growth, differentiation of cells, inhibition of cell death or another desired product.

The reservoir 7, 7' is held in position through the forces of surface tension. If necessary, it is possible to add a holding means, such as a surgical adhesive or any other equivalent means to enhance the attachment of the reservoir 7, 7. The choice of a retaining means may be linked to the application in question.

The advantage of the invention is to apply a medium 3 for example a suspension in a localized and precise manner. As indicated above, the lens, and more generally the carrier 1 used to apply the medium 3 may include one cavity 2, or more than one cavity 2, which is (are) located at the selected/required place for applying said medium.

In addition, the use of channels 6 allows refilling of the microcavities 2 either directly or by using the additional reservoir 7.

In the case of the presence of several cavities 2, they may be supplied by the same medium 3 or different media 3 for different effects and/or complementary effects or by any other desired product.

The reservoirs 7, 7' as illustrated in figures 8 and 9 may be used in a configuration of a single cavity 2 or multiple cavities 2 as illustrated in figures 5 and 5A. In such case, a reservoir 7/7' may be used to supply all cavities 2 with the same medium 3, or the reservoir 7/7' may supply only a certain number of cavities 2. In another configuration, the reservoir 7/7' may comprise several separations each containing a different medium 3 for a selected supply of different medium 3 to different cavities 2.

When changing the reservoir 7 according to the procedure illustrated in figure 9, the replacement reservoir 7' may supply the same cavities 2 than replaced reservoir 7 with the same medium 3 or a different one, or certain cavities only with the same medium 3 or a different one. Depending on the shape of the reservoirs 7/7' and the presence of separations with different media 3, they may also supply different cavities (not the same ones) to vary the location of application of medium 3 and/or the medium 3 being applied. As one skilled in the art will understand, the principle of the invention allows many variants that may be tailored to the intended use/application of the invention.

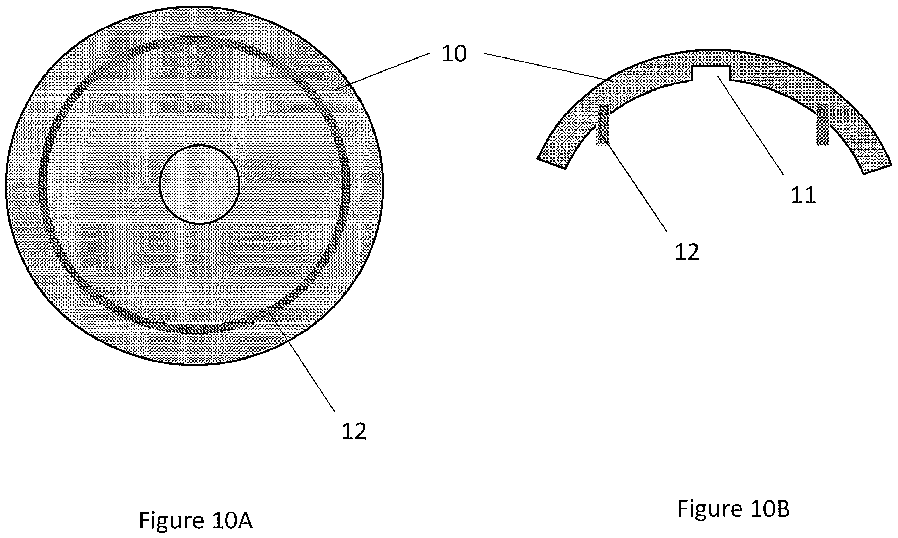

Figures 10A (bottom view) and 10B (side cut view) illustrate another embodiment of a carrier for example a lens 10. In this embodiment, the lens 10 comprises a ring 12 which surrounds the cavity 11 (corresponding to the cavity 2 described above). The ring 12 is used to "protect" the medium 3 present in said cavity 11 from native cells so that the freshly transplanted cells (when the medium is a cell suspension) can properly cover the treated zone of the patient.

The ring 12 may be in any suitable material for the intended application of the device (not limited to a lens). Preferably the material is a biocompatible one, for example a metal or a synthetic material.

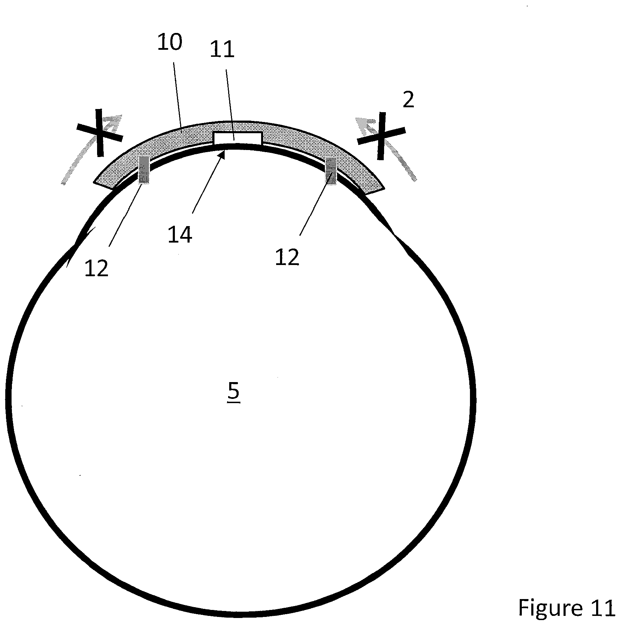

Figure 11 (cut view) illustrates this feature with a lens 10 applied to an eye 5 of a patient, the crossed arrows showing the protection provided by the ring to the treated zone 14 of the eye 5.

Taking advantage of the fact that the device may comprise more than one cavity 11 (2) the device can be functionalized to improve homogenous and rapid coverage of the grafted area of a patient by appropriate arrangement of the cavities 11/2 on the carrier 1/10.

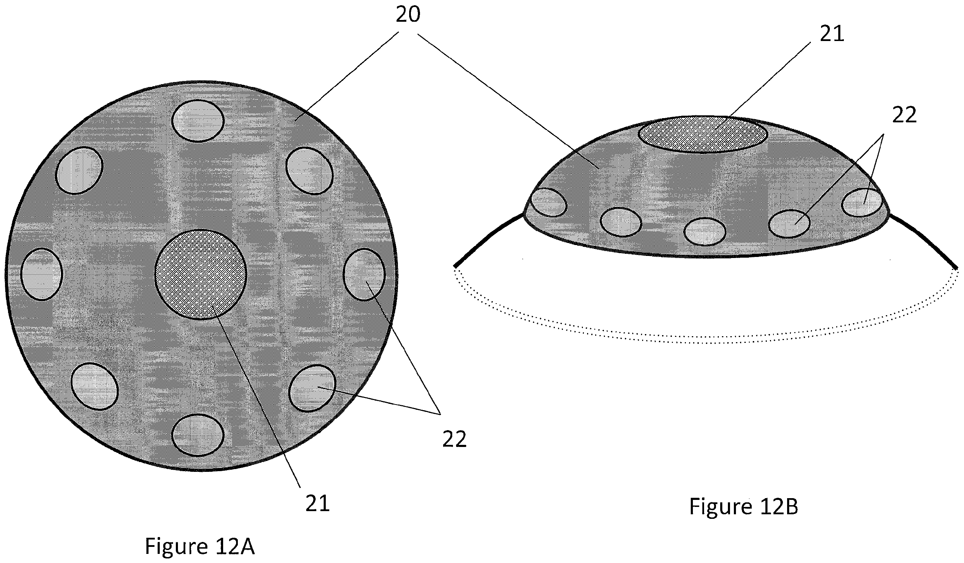

Accordingly, as also described hereabove and applicable to said embodiments, the device may comprise in one embodiment a central cavity 11 containing the medium 3, for example cells to be transplanted and more external cavities 11 forming an outer ring containing growth factors of chemo attractants that will diffuse through the stroma. Thus the grafted cells will be attracted to the periphery and will cover homogenously and rapidly the area to be healed. This embodiment is illustrated in figures 12A and 12B where the carrier in the shape of a contact lens 20 comprises a central cavity 21 which is surrounded by several cavities 22 which may contain the appropriate substances in accordance with the principle of the invention. The surrounding cavities 22 may contain any suitable media 3 to obtain the desired effect. They may also be used to provide an effect equivalent ("shielding effect") to the one obtained with the ring 12 of figures 10 and 11 (see above).

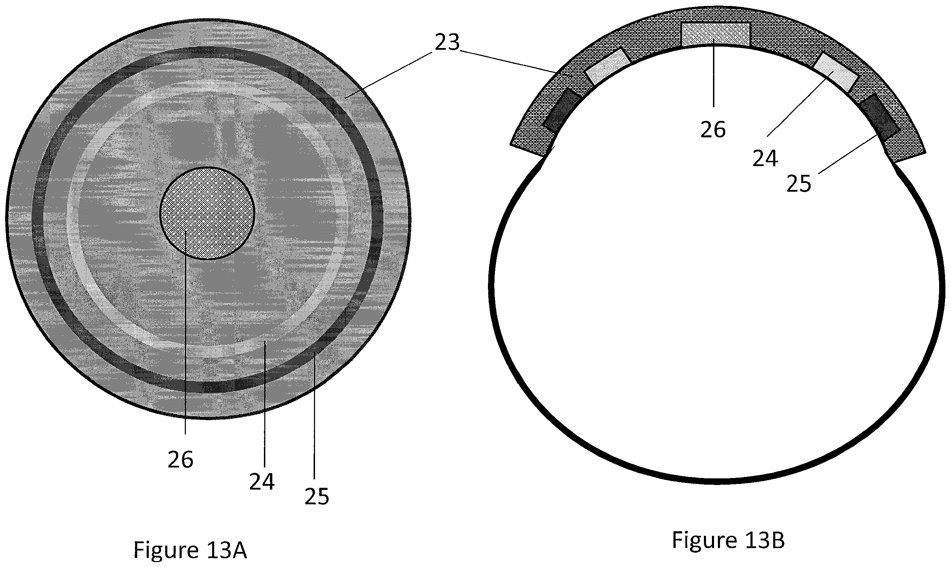

In another embodiment illustrated in figures 13A (bottom view) and 13B (side cut view), the carrier 23 may be built as described above with the addition of several more ring shaped cavities 24, 25 containing for example growth factors, small molecules promoting proliferation or a growth inhibitor. Indeed, the grafted cells of the cavity 26 need to be protected from native cells during the early time after transplantation. Thus the growth inhibitor (for example in ring 25) will stop the native cells from invading the transplantation area. But, thanks to the ring 24 containing growth factors or promoting molecules, the growth of transplanted cells will not be inhibited. In an embodiment, the outside ring 25 may contain inhibitors of blood vessels growth to prevent neovascularization that often occur when the cornea is damaged.

In the case of transplantation in a severely damaged environment the transplanted cells not only need to grow and cover the wounded area but they firstly need to survive. To answer this need, the device carrier may comprise some cavities containing cells, some other containing growth factors, small molecules promoting proliferation and some containing cell death inhibitors etc. As one will readily understand from the above description, the present invention allows many different configurations of carrier with cavities and media being applied to a patient.

In some embodiments, the carrier may be built with numerous cavities 2 in such a way to create gradients of media 3 being applied such as cell suspension, growth factor, cell death inhibitors etc....

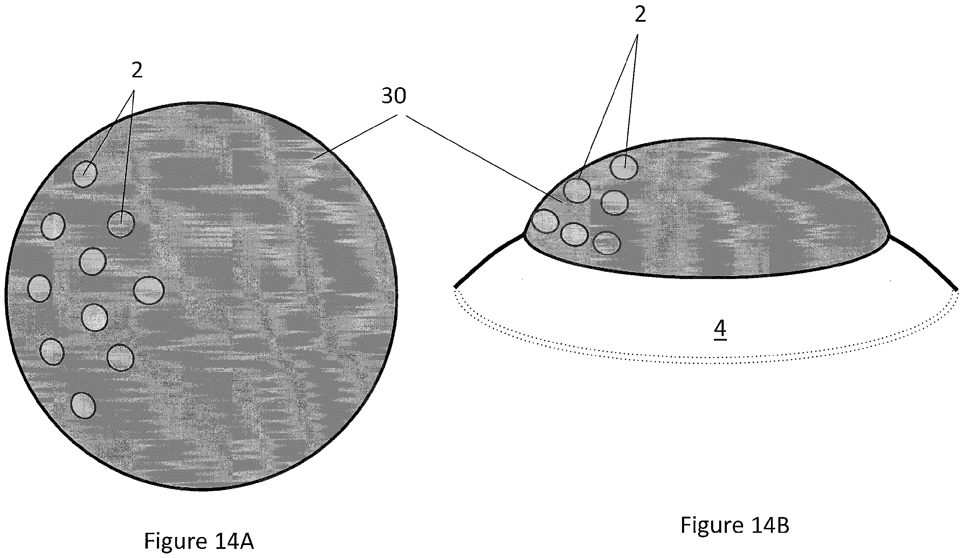

This gradient may be achieved by different constructions illustrated in figures 14-16.

In the embodiment of figures 14A (bottom view) and 14B (side view), the carrier 30 (for example a lens) comprises a plurality of cavities 2 which are arranged asymmetrically on said carrier 30, on one side of it. The medium 3 carried in said cavities 2 may be the same in all cavities 2 or different and may diffuse underneath the carrier 30 (to the right in figures 14A and 14B). The concentration of media will however be the highest where the cavities 2 are located, the concentration being also influenced by the volume of the cavities which may be the same or different.

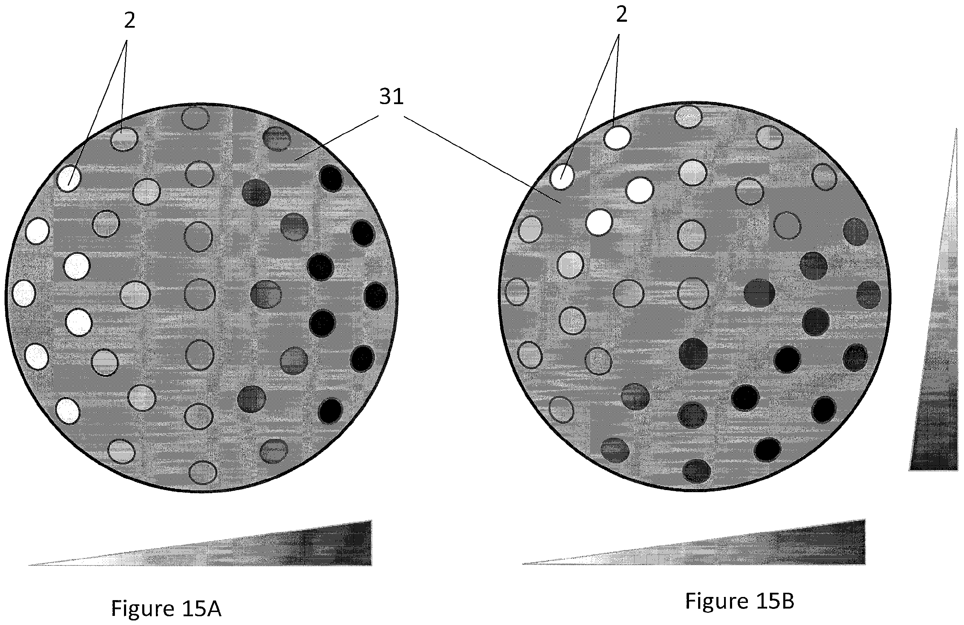

In the embodiment of figures 15A to 15CB, another version is shown which applies a gradient of concentration of medium 3 via the cavities 2 of the carrier 31. Figure 15A illustrates a 1D gradient: the concentration increases from the left side to the right side (from brighter to darker). This gradient may be achieved by increase of the density of the cavities 2 and/or increase of their volume, or an increase of concentration of medium or a combination thereof.

In figure 15B, the gradient has 2 dimensions created by the cavities 2. As for the embodiment of figure 15A, the increase of concentration may be achieved by increase of the density of the cavities 2 and/or increase of their volume and/or increase of concentration of medium or a combination thereof.

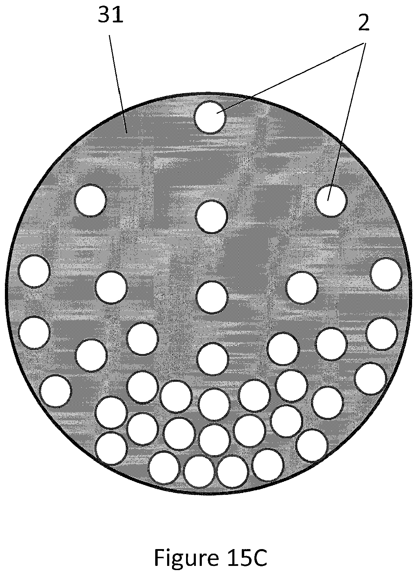

In figure 15C, an embodiment is illustrated where the increase of density of cavities 2 is shown in the lower part of the carrier 31.

An advantage of using an increase of density of cavities 2 or of the cavitie's volume rather than to increase the concentration of the medium is that there is no need to change the medium over time to achieve this effect. Of course, in certain circumstances it may be better to change the concentration of the medium.

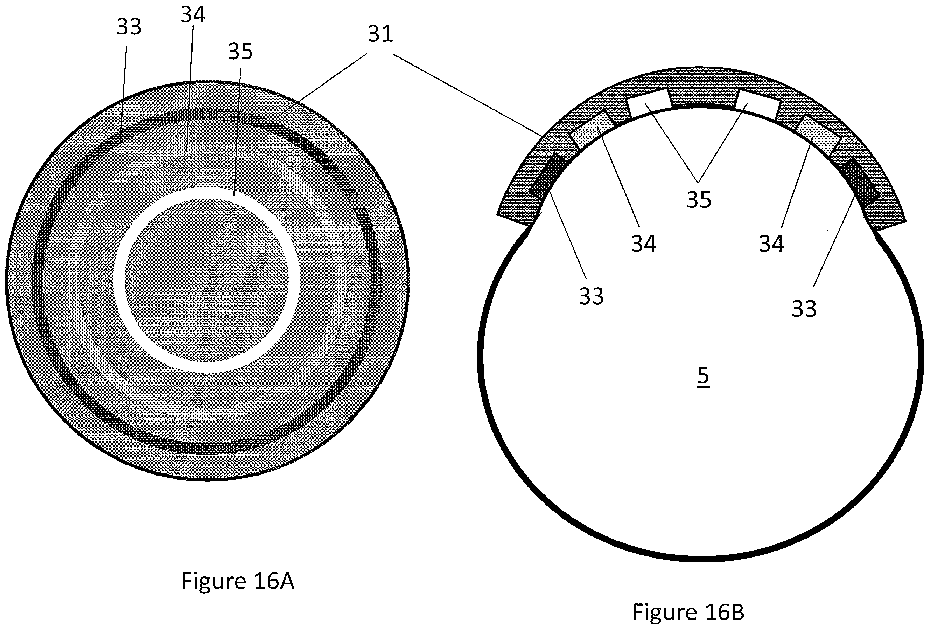

In figures 16A (bottom view) and 16B (side cut view), the cavities of the carrier 32 have the shape of rings 33, 34, 35 and such rings 33-25 may also be used to create a concentration gradient of the medium. Each ring 33-35 may carry the same medium or a different one and the gradient may be achieved by the shape and volume of the ring.

It is of course understood that when using several different chemical substances for the creation of gradients in the carrier, the gradients of the individual substances can be controlled individually and hence combined arbitrarily (same, opposing or arbitrarily different directions and geometries).A skilled person will recognize the many combination possible with the principle of the invention and the combination of features of embodiments.

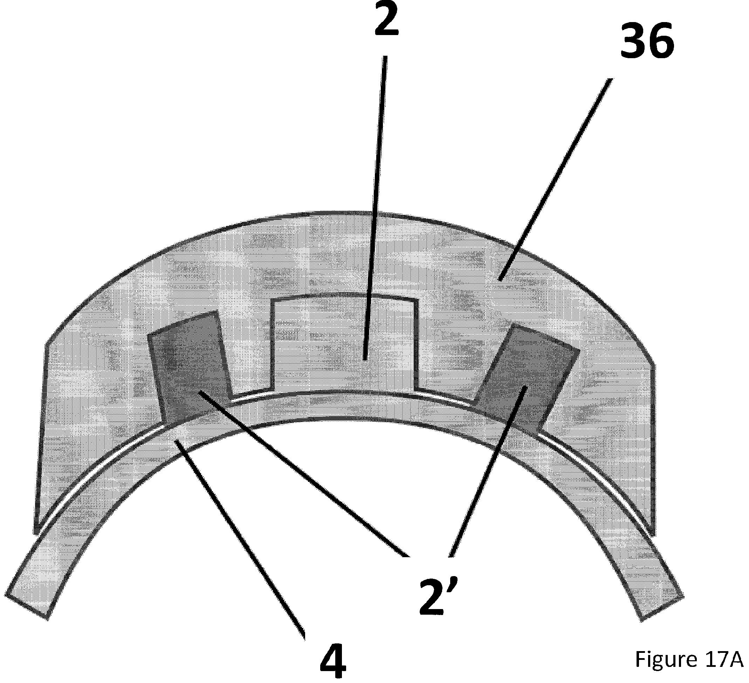

Figures 17A to 17C (side cut views) illustrate a further example useful for a particular application: permeabilization or local removal of the surface epithelium of the target organ can be desired. Transplantation of cells to deeper layers is an example, as is treatment of deeper layers by chemical substances. The simplest way of achieving this is to mechanically or chemically remove part or all of the surface epithelium before device placement.

In the case of chemical permeabilization, the carrier 36 (for example a contact lens) comprises at least one cavity 2 for cells in suspension as a medium 3, and other cavities (in this example two cavities 2') which form distinct permeabilization cavities 2, 2', the surface epithelium can be removed or permeabilized in the vicinity of the cavities 2' containing the permeabilization agent, eventually allowing the cells in the main cavity 2 to reach the interior of the cornea 4 of the eye 5.

Taking advantage of the localized action provided by microcavities 2, 2', one can treat the corneal surface epithelium locally by conditions that result in the local removal of the surface epithelium. This can be achieved by means of application of solutions of permeabilizing agents in at least some of the cavities 2'. These permeabilizing agents can be tensides (such as benzalkonium salts), enzymes (such as Trypsin or collagenases), chelating agents (such EDTA), photodynamic agents (such as gamma-aminolevulinic acid and derivatives which are processed to photoactive porphyrins by the cells) as well as combinations of these agents. The permeabilization allows transplantion of cells directly to the surface of the stroma. It is possible to use hydrogels in the permeabilization cavities to avoid unintended mixing of the cells (medium 3) and the permeabilization agent prior to transplantation.

Figure 17A illustrates a lens 36 able to do both treatments: permeabilization and treatment per se.

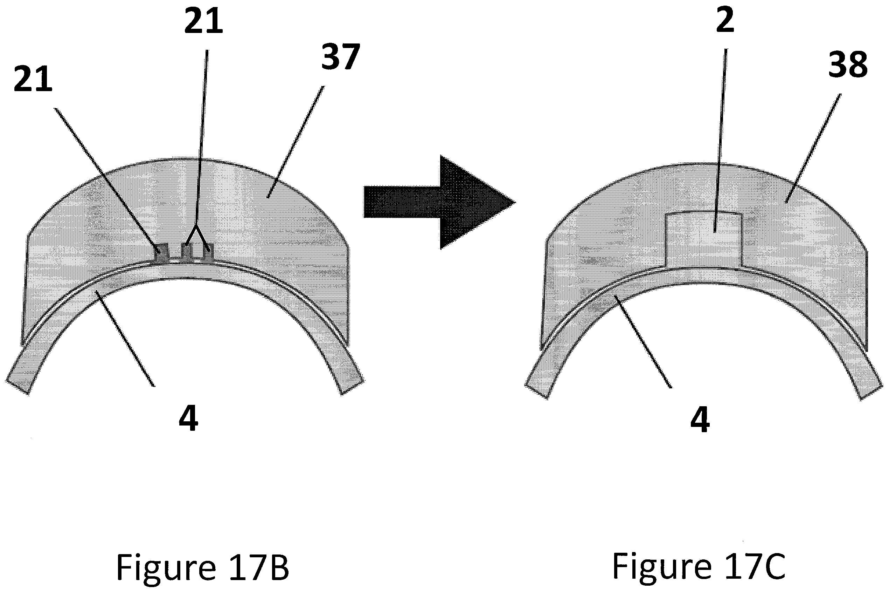

Figures 17B and 17C illustrate the same process but in two steps with two different carriers 37, 38 the carrier 37 being used for the permeabilization step with cavities 2' containing the permeabilization agent and the carrier 38 for the treatment step (for example a cell transplantation) with cavity 2 containing the medium 3.

Of course, all the teachings and configurations of previous embodiments described above may be applied here to the embodiments of figures 17A - 17C.

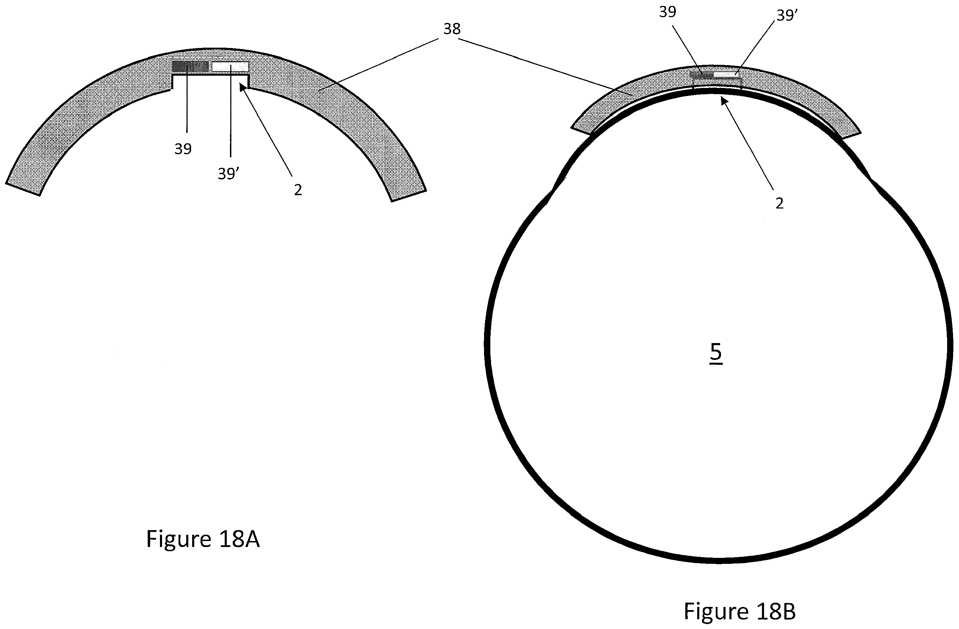

Figures 18A (side cut view) and 18B (side cut view on the eye 5 of a patient) illustrate another embodiment of a carrier 38 in which a sensor 39, 39' (for example a micro-sensor) is placed next to the cavity 2. The sensor is typically intended to measure or monitor different parameters, for example to monitor PH, Co2, and metabolites concentration. This may of course be adapted depending on the application of the invention, when different applications need different parameters to be monitored and the carrier may comprise more than one sensor 39, 39'.

For example, the carrier 38 may be provided with sensors 39, 39' allowing quantifying different aspects of the cellular metabolism and growth transplantation. For instance, pH, carbon dioxide, oxygen or glucose concentration may be used to define the time points when a medium change is needed. The sensors 39, 39' may use any operating principle compatible with the spatial restraints, but typically electrical or optical mechanisms may be used.

For instance, microelectrodes 39, 39' embedded in the carrier and reaching the medium may be used for sensing pH, glucose or oxygen; readout systems for electrical measurements embedded in contact lenses are available as well.

In another variant, optical sensing is based on substances which change optical properties as a function of an analyte concentration.

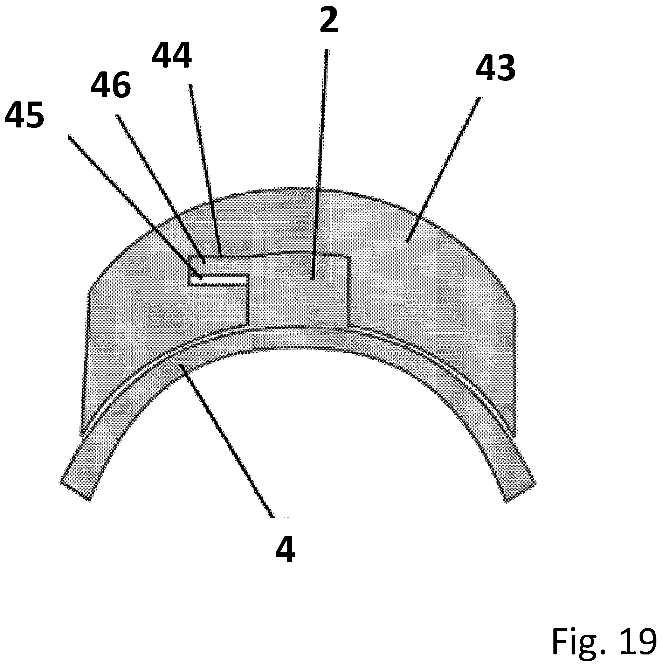

Figure 19 illustrates an embodiment of a carrier having at least one sensor wherein the sensor may be made of a dye indicator contained in the medium 3 carried in the cavity 2. Accordingly, the carrier 43 (for example a contact lens) comprises a cavity 2 with a medium 3 to be applied to a patient on the cornea 4, the cavity comprising a side extension 44 which is used to allow reading of the dye reaction. To this effect, the carrier 43 comprises an optical background 45 suitable for an optical readout.

In an embodiment, the side extension 44 comprises an optical background 45 and also a polymer or hydrogel pouch 46 containing indicator substances for the reaction.

Of course, as mentioned, a carrier may comprise one single sensor or a plurality of sensors of the same type or of a different type to detect/monitor different variables and elements, according to circumstances. Many variants are thus possible in the frame of the present invention.

An example of a readout system that may be used in the present context for electrical sensing is the one developed by the Company named Sensimed (www.sensimed.ch), as disclosed in the application WO 03/001991. In particular, one may combine the signal processing and readout systems described therein with microelectrodes measuring metabolites, oxygen tension, pH or carbon dioxide concentrations. "

The result of the sensing may be used to determine whether the medium 3 has to be resupplied and/or exchanged and/or adapted in accordance with a result sought. Such refilling, exchange and/or adaptation may be carried out with the embodiments of the invention described hereunder.

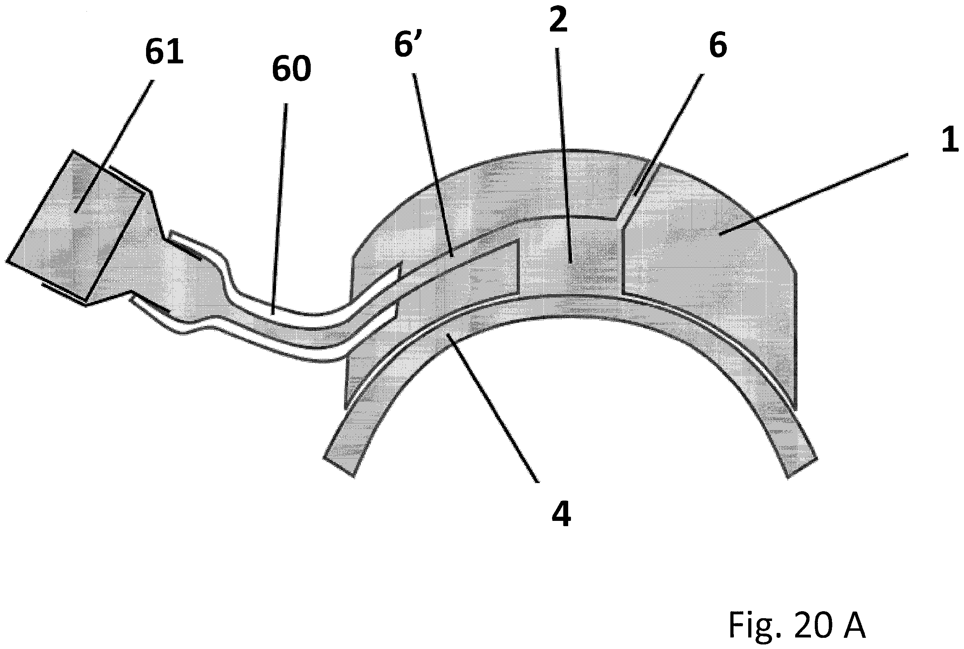

Figures 20A-20D illustrates examples with a medium supply/refilling capability and the procedure therefor.

Upon transplantation, it is important that the cells are in a controlled environment. Several solutions are possible to provide the cells with nutrients for a time period suitable for successful implantation (typically, about 10 days) prior to exposure to the native environment when removing the device. Possible solutions are the following:
-) Post-transplantation lens, a lens which is regularly replaced with a fresh device or at least fresh medium, for example by a reservoir as described above in relation to figures 8 and 9;
-) Post-transplantation or transplantation lens containing either a medium concentrate or solid powder in a specific reservoir, covered with a water permeable membrane that allows reconstitution of the medium to near isotonic concentration by diffusion of water from the tear film to the concentrate and slow release into the main chamber through a connection channel; an outlet channel can be used to release the spent medium into the tear film.
-) Alternatively, a transplantation or post-transplantation lens with micro-channels and attached tubing allowing for liquid replenishment. For sterility, a sterile fluid injection based on a rubber septum, which accepts multiple injections with hypodermic needles, may be used; also valves that only open when a threshold pressure is reached can be placed in the outlet channel to avoid infection of the main chamber;
-) A further variant comprises valves that open when the pressure in the reservoir channel reaches a threshold level. Another such pressure-sensitive valve can be placed in the outlet channel to limit infection.

Possible embodiments and variants are described in more detail hereunder.

In figure 20A, the carrier 1 (for example a contact lens) comprises a cavity 2 intended to contain a medium 3 for the treatment of a patient (for example the cornea of the patient) in accordance with the principle of the present invention. As described above in relation to figures 4-9, the carrier 1 comprises channels 6, 6' for the supply of the medium 3. Typically, the carrier 1 comprises an outlet 6 (to allow evacuation of cavity 2) and an inlet 6' for the supply of the desired medium 3. The device used to this effect illustrated in figure 20A comprises a tubing 60 to be connected to inlet 6' and an injection port 61 allowing the transfer of medium into the cavity 2 when the carrier 1 is in place. This is an example of an external injection/supply method.

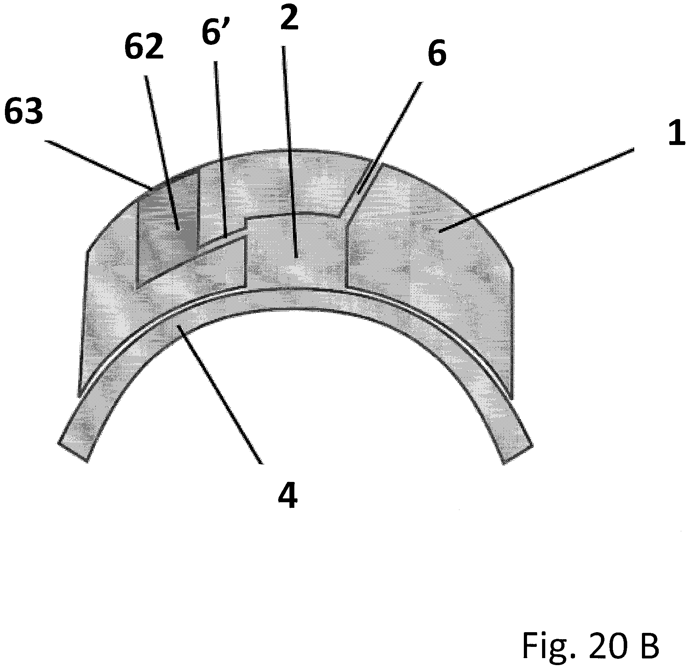

In figure 20B, the process is to avoid an external supply but rather to reconstitute a medium using the diffusion of water from the tear film of a patient. The carrier 1, such as a contact lens, has a previously disclosed a cavity 2 for the medium 3 to be applied to the cornea 4 of a patient. The carrier comprises an outlet 6 and an inlet 6' of the supply of the cavity. In this embodiment, the inlet 6' is connected to a medium concentrate 62 covered by a water permeable membrane 63 through which water from the tear film may diffuse into the concentrate 62 and thereby reconstruct the supply of medium 3 in the cavity 2.

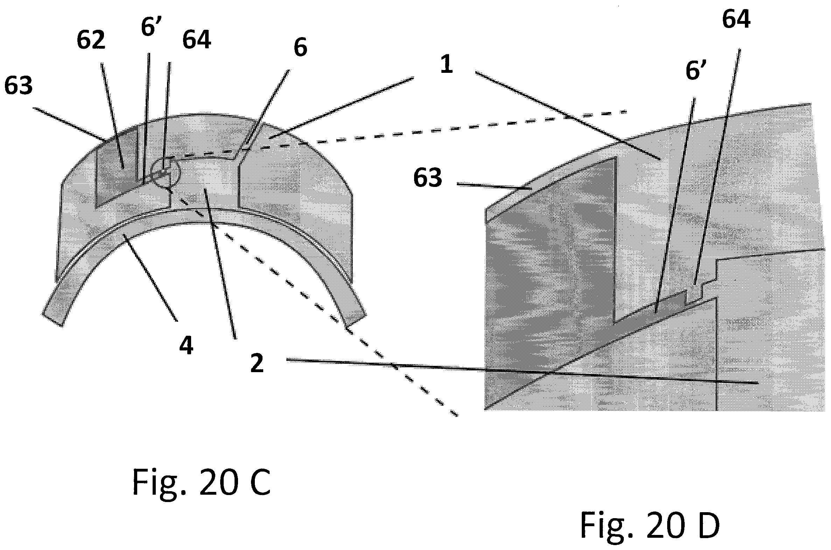

Figures 20C and 20D illustrate another variant of the embodiment of figure 20B. In this variant, the same elements are identified by the same references as in figure 20B. In addition, this variant features a valve 64 placed in the inlet 6' that requires a slight overpressure in the chamber with the medium concentrate 62. Since it takes time to build up this overpressure upon diffusion of water from the tear film through the water permeable membrane 63 to the medium concentrate chamber 62, release of reconstituted medium into the cavity 2 is delayed and occurs in a more controlled way.

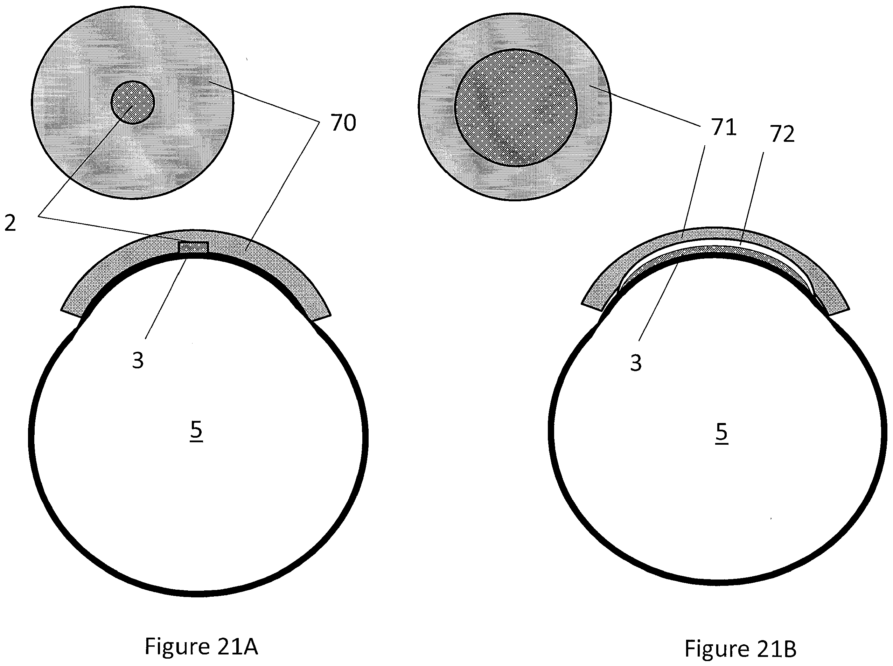

Figures 21A (top and side cut views), 21B (top and side cut views), 22A (bottom and side cut views), 22B (bottom and side cut views) and 22C (bottom and side cut views) illustrate an example of a method.

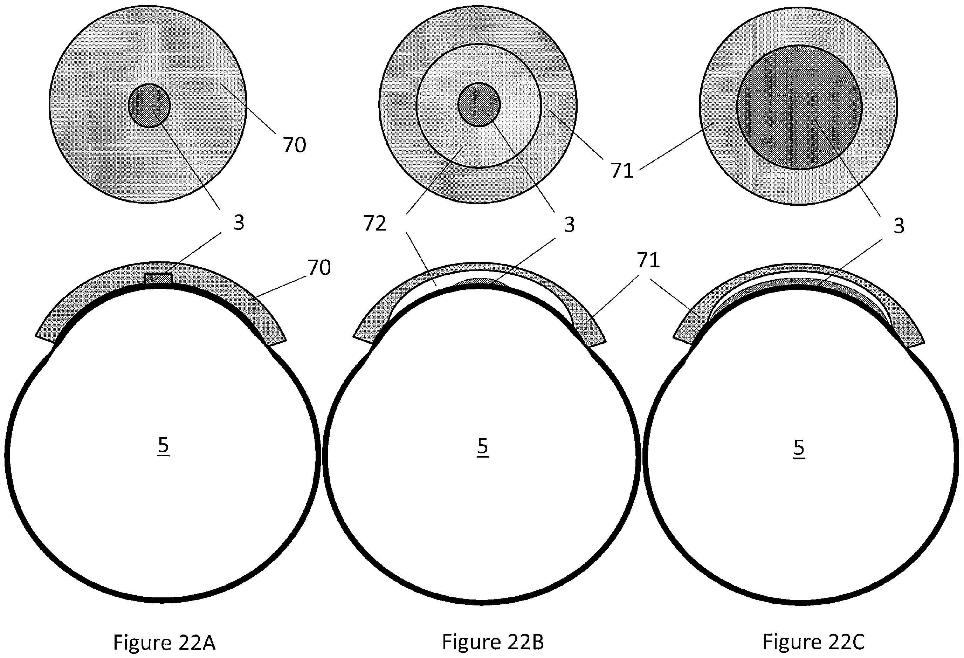

Taking advantage of the fact that the size of device's cavity 2 can be chosen, as described hereabove in relation to the different embodiments of the invention, one may construct two different carriers, i.e. lenses: a first seeding lens 70 (corresponding to lens 1) with the appropriate cavity 2 volume to seed the desired amount of cells (as the medium 3), and a second lens 71 with a larger cavity 72 to allow for a good cell 3 growth. The transplantation procedure can be the following:
First step (figures 21A and 22A): transplantation of the cells by applying the seeding lens 70 containing the cells suspension 3 for the needed time (typically 2 to 3 hours).
Second step (figure 22B): the seeding lens 70 is removed and a lens 71 with a larger cavity 72 (containing cell culture medium) is placed on top of the transplanted cells 3. Thus this second lens 71 will allow for cell growth and will protect the transplanted cells (which are quite fragile during the first time of transplantation) from the shear forces dues to intraocular movements, see figures 21B and 22C.

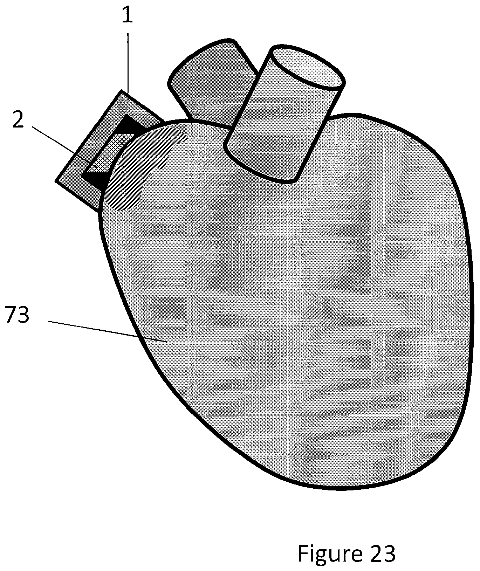

Figure 23 illustrates another example showing the same kind of device described above in the present application being adapted to other organs: skin, scalp, or internal organs such as heart 73 (illustrated in figure 23) etc...Although the present description mainly refers to the construction of a lens as a carrier, this should not be regarded as limiting the scope of application of the present invention which may be used in other shapes or forms to treat other parts of the body of a patient in accordance with the principle of the present invention.

### Envisaged Applications

Applications of the present invention are numerous. As mentioned earlier, one may use this support in the form of contact lens but this should not be construed as a limitation of the present invention.

The preferred application of device according to the present invention is the use of a contact lense as described to transplant corneal epithelial stem cells. Indeed, many patients suffer from deficient vision due to damaged (chemical or thermic burns) or absent (limbal stem cell deficiency) corneal epithelium. Today, these conditions are treated by transplantation of cornea or limbal grafts. Transplantation of cells in suspension, thanks to the device described herein, facilitates and accelerates the transplantation procedure for the surgeon. Moreover, the technique described herein makes creation of an artificial epithelium in culture under expensive GMP conditions superfluous. Indeed, the surgeon can directly transplant cells obtained from the healthy contralateral eye, or a donor eye. Accordingly, the use of the present invention in this application is particularly advantageous.

Applications of the present invention are however numerous. Examples include:
-) It can be used to transplant genetically modified cells (for example for the correction of heritable corneal dystrophies) or non-epithelial cells (stromal, multipotent etc) to the ocular surface;
-) Transplantation of cells in suspension to other superficial tissues, e.g. gums, mucuous membranes, skin, for instance for hair follicle regeneration
-) Dosable application of substances with or to the grafted cells in order to improve engraftment, proliferation and differenciation of cells

The device of the present invention may potentially be used in a variety of other applications on the eye. For instance one can transplant genetically modified cells (for example for the correction of heritable corneal dystrophies) or non-epithelial cells (stromal, multipotent etc).

These applications may of course be combined among them.

With some adaptation, the device can be used for internal organs as well as mentioned earlier in the present application and illustrated in figure 23 schematically:
The device generally has to be fixed to the organ surface by a mechanical method. Most commonly, this will be surgical sutures, fibrin glue or some form of mechanical bandage.

The principle of the present invention is still used in other applications and with different carriers as described below.

For example:
-) Transplantation of cells in suspension This can be realized on different tissues: tissue or organ such as eyes, gums, skin.
-) a contact lens for cell transplantation on the ocular surface or a device for transplantation of buldge stem cells for hair follicles regeneration.
-) Dosable application of substance in grafted cells in order to improve engraftment, proliferation and differenciation of cells

These applications may of course be combined among them.

Permabilization for transplantation to deep layers may be employed as needed (see the above description of this embodiment).

The size of the device, and volume capacity of the permeabilization and cell cavities should be adapted to the organ at hand and to the desired application.

Depending on the surgical procedure, it may be necessary to temporarily immobilize the cells, for example by a spontaneously liquefying hydrogel (e.g. surgical fibrin gels + plasmin or ca-alginate gels + alginate lyase).

Further example of target applications include among others: transplantation of genetically modified autologous hepatocytes to the superficial layers of the liver for correction of inborn errors of hepatic metabolism; transplantation of hormone-producing cells into the dermis for correction of endocrine diseases; transplantation of genetically corrected muscle satellite cells to muscle tissue for the treatment of heritable muscular dystrophies; application of immune cells to solid tumors for cancer therapy.

Any suitable material (including biocompatible) may be used to form the support.

In the case of the lens, for example be used and a method of the PDMS mold.

One may also use flexible silicone or other suitable material depending on the application.

In one embodiment, one may use a lens that is adjustable to light ("light adjustable lens," Calhoun Vision LAL, Pasadena, Calif.), in which the power or correction of the lens can be adjusted after implantation in the eye.

The inner surface of the support, that is to say one that will be in contact with the user/patient or animal (e.g. in the case of eye lens) may receive a specific treatment such as adding a surgical glue or other.

### Technical and economic advantages

The transplantation technique described herein allows the use of cells in suspension. One therefore avoids a cell culture on a lens or another media as proposed in other known devices. This makes transplanting easier and faster.

This device has the advantage of having pockets/cavities of small to large sizes the location of which may be chosen. This allows the control of the location and of the quantity of cells applied to the target tissue.

The realization of the device is done directly by molding, a simple process that does not necessitates reworking.

As mentioned above, the principle according to the invention may be used in many applications and may be designed for application to human tissue or a human organ, such as the eye, gums, skin, injury or burns etc ... This device may be useful for veterinary use in similar applications.

The described embodiments allow many different constructions in accordance with the principles detailed above. The invention is not limited to a use with a lens but many different applications may be envisaged with an appropriate carrier/support suitable for the intended use or application.

The size, shape and volume of the cavities may be varied according to circumstances and/or to adapt to the intended use and application, the aim being to apply the desired medium at the desired location in the desired concentration to achieve the desired effect. The disposition of the cavities may be symmetrical or asymmetrical.

## Claims

1. A device for a direct application of a medium (3) in the form of a cell suspension, on tissue of a patient, said device comprising a carrier (1) to be applied to said tissue with at least one micro-cavity (2) for containing said medium, said micro-cavity being in direct contact with the tissue on which the said medium is to be applied for a targeted application of said medium, said device comprising at least one channel (6,6') for the supply of the at least one micro-cavity (2) with said medium (3) and at least one reservoir (7, 7') which allows a continuous feeding of the at least one micro-cavity (2) with medium (3) over time through said at least one channel (6,6').

2. The device according to claim 1, wherein the carrier (1) comprises several micro-cavities (2).

3. The device according to one of the preceding claims, wherein the carrier (1) has the shape of a contact lens.

4. The device according to one of the preceding claims, wherein the carrier comprises a plurality of cavities (2) that are evenly distributed on the carrier (1) or which are not evenly distributed on the carrier (1).

5. The device according to one of the preceding claims, wherein the carrier (10) comprises protection means (12) to protect the medium from the environment when the carrier is applied to a patient.

6. The device as defined in the preceding claim, wherein the protection means is a ring (12) or a plurality of cavities (22).

7. The device as defined in one of the preceding claims, wherein the cavity (2) or the cavities (2;21;22) have a cylindrical shape, and/or a ring shape (24,25) and/or have different volumes.

8. The device as defined in one of the preceding claims, wherein the device comprises a sensor (37, 37').

9. The device as defined in the preceding claim, wherein the sensor is placed in the carrier (1).

10. The device as defined in one of claims 8 or 9, wherein the sensor an indicator dye in the medium (3) or microelectrodes.

11. The device as defined in one of claims 8 to 10, wherein the sensor comprises a polymer or hydrogel pouch (46).

12. The device as defined in one of the preceding claims, wherein in order to reconstitute the medium (3) the carrier comprises at least an inlet (6') connected to a medium concentrate chamber (62) covered by a water permeable membrane (63).

13. The device as defined in the preceding claim, wherein the inlet (6') further comprises a valve (64) to build an overpressure in the chamber (62).

14. The device as defined in one of the preceding claims, wherein it comprises a plurality of cavities (2), wherein some cavities contain the medium, and other cavities contain a growth inhibitor, and/or a growth factor, and/or inhibitors of blood vessels, and/or cell death inhibitors and/or a permeabilization agent.

15. The device as defined in one of claims 8 to 14, wherein the sensor is a pH, CO2 or metabolite concentration sensor.

16. The device according to one of the preceding claims wherein the carrier is adapted for application to human tissue or human or animal body, such as the eye, the gingiva, skin, a wound or a burn or an internal organ.

## Patentansprüche

1. Vorrichtung zur direkten Applikation eines Mediums (3) in Form einer Zellsuspension auf Gewebe eines Patienten, wobei die Vorrichtung einen Träger (1), der auf das Gewebe zu applizieren ist, mit mindestens einem Mikrohohlraum (2) zum Enthalten des Mediums umfasst, wobei sich der Mikrohohlraum in direktem Kontakt mit dem Gewebe befindet, auf welches das Medium zur gezielten Applikation des Mediums zu applizieren ist, wobei die Vorrichtung mindestens einen Kanal (6, 6'), um dem mindestens einen Mikrohohlraums (2) das Medium (3) zuzuführen, und mindestens ein Reservoir (7, 7') umfasst, welches ein kontinuierliches Speisen des mindestens einen Mikrohohlraums (2) mit Medium (3) im Zeitverlauf durch den mindestens einen Kanal (6, 6') ermöglicht.

2. Vorrichtung nach Anspruch 1, wobei der Träger (1) mehrere Mikrohohlräume (2) umfasst.

3. Vorrichtung nach einem der vorhergehenden Ansprüche, wobei der Träger (1) die Form einer Kontaktlinse hat.

4. Vorrichtung nach einem der vorhergehenden Ansprüche, wobei der Träger eine Vielzahl von Hohlräumen (2) umfasst, die gleichmäßig auf dem Träger (1) verteilt sind oder die nicht gleichmäßig auf dem Träger (1) verteilt sind.

5. Vorrichtung nach einem der vorhergehenden Ansprüche, wobei der Träger (10) Schutzmittel (12) umfasst, um das Medium vor der Umgebung zu schützen, wenn der Träger einem Patienten appliziert wird.

6. Vorrichtung nach dem vorhergehenden Anspruch, wobei das Schutzmittel ein Ring (12) oder eine Vielzahl von Hohlräumen (22) ist.

7. Vorrichtung nach einem der vorhergehenden Ansprüche, wobei der Hohlraum (2) oder die Hohlräume (2; 21; 22) eine zylindrische Form und/oder eine Ringform (24, 25) hat bzw. haben und/oder unterschiedliche Volumina hat bzw. haben.

8. Vorrichtung nach einem der vorhergehenden Ansprüche, wobei die Vorrichtung einen Sensor (37, 37') umfasst.

9. Vorrichtung nach dem vorhergehenden Anspruch, wobei der Sensor in dem Träger (1) platziert ist.

10. Vorrichtung nach einem der Ansprüche 8 oder 9, wobei der Sensor ein Indikatorfarbstoff in dem Medium (3) oder Mikroelektroden ist.

11. Vorrichtung nach einem der Ansprüche 8 bis 10, wobei der Sensor einen Polymer- oder Hydrogelbeutel (46) umfasst.

12. Vorrichtung nach einem der vorhergehenden Ansprüche, wobei der Träger, um das Medium (3) zu rekonstituieren, mindestens einen Einlass (6') umfasst, der mit einer Mediumkonzentratkammer (62) verbunden ist, die von einer wasserpermeablen Membran (63) bedeckt ist.

13. Vorrichtung nach dem vorhergehenden Anspruch, wobei der Einlass (6') des Weiteren ein Ventil (64) umfasst, um einen Überdruck in der Kammer (62) aufzubauen.

14. Vorrichtung nach einem der vorhergehenden Ansprüche, die eine Vielzahl von Hohlräumen (2) umfasst, wobei einige Hohlräume das Medium enthalten, und andere Hohlräume einen Wachstumsinhibitor und/oder einen Wachstumsfaktor und/oder Inhibitoren von Blutgefäßen und/oder Zelltodinhibitoren und/oder ein Permeabilisierungsmittel enthalten.

15. Vorrichtung nach einem der Ansprüche 8 bis 14, wobei der Sensor ein pH-, CO₂- oder Metabolitenkonzentrationssensor ist.

16. Vorrichtung nach einem der vorhergehenden Ansprüche, wobei der Träger zur Applikation auf menschliches Gewebe oder den menschlichen oder tierischen Körper, wie das Auge, die Gingiva, die Haut, eine Wunde oder eine Verbrennung oder ein inneres Organ vorgesehen ist.

## Revendications

1. Dispositif pour une application directe d'un milieu (3) sous la forme d'une suspension cellulaire, sur le tissu d'un patient, ledit dispositif comprenant un support (1) à appliquer sur ledit tissu avec au moins une micro-cavité (2) pour contenir ledit milieu, ladite micro-cavité étant en contact direct avec le tissu sur lequel ledit milieu doit être appliqué pour une application ciblée dudit milieu, ledit dispositif comprenant au moins un canal (6, 6') pour l'alimentation de l'au moins une micro-cavité (2) avec ledit milieu (3) et au moins un réservoir (7, 7') qui permet une alimentation continue de l'au moins une micro-cavité (2) avec le milieu (3) au fil du temps à travers ledit au moins un canal (6, 6').

2. Dispositif selon la revendication 1, le support (1) comprenant plusieurs micro-cavités (2).

3. Dispositif selon l'une des revendications précédentes, le support (1) ayant la forme d'une lentille de contact.

4. Dispositif selon l'une des revendications précédentes, le support comprenant une pluralité de cavités (2) qui sont réparties de manière homogène sur le support (1) ou qui ne sont pas réparties de manière homogène sur le support (1).

5. Dispositif selon l'une des revendications précédentes, le support (10) comprenant des moyens de protection (12) pour protéger le milieu de l'environnement lorsque le support est appliqué sur un patient.

6. Dispositif selon la revendication précédente, le moyen de protection étant un anneau (12) ou une pluralité de cavités (22).

7. Dispositif selon l'une des revendications précédentes, la cavité (2) ou les cavités (2 ; 21 ; 22) ayant une forme cylindrique, et/ou une forme d'anneau (24, 25) et/ou ayant des volumes différents.

8. Dispositif selon l'une des revendications précédentes, le dispositif comprenant un capteur (37, 37').

9. Dispositif selon la revendication précédente, le capteur étant placé dans le support (1).

10. Dispositif selon l'une des revendications 8 ou 9, le capteur étant un colorant indicateur dans le milieu (3) ou des microélectrodes.

11. Dispositif selon l'une des revendications 8 à 10, le capteur comprenant une poche (46) en polymère ou en hydrogel.

12. Dispositif selon l'une des revendications précédentes, pour reconstituer le milieu (3), le support comprenant au moins une entrée (6') reliée à une chambre de concentration du milieu (62) recouverte d'une membrane perméable à l'eau (63).

13. Dispositif selon la revendication précédente, l'entrée (6') comprenant en outre une vanne (64) pour construire une surpression dans la chambre (62).

14. Dispositif selon l'une des revendications précédentes, celui-ci comprenant une pluralité de cavités (2), certaines cavités contenant le milieu, et d'autres cavités contenant un inhibiteur de croissance, et/ou un facteur de croissance, et/ou des inhibiteurs de vaisseaux sanguins, et/ou des inhibiteurs de mort cellulaire et/ou un agent de perméabilisation.

15. Dispositif selon l'une des revendications 8 à 14, le capteur étant un capteur de pH, de CO₂ ou de concentration de métabolites.

16. Dispositif selon l'une des revendications précédentes, le support étant adapté pour être appliqué sur un tissu humain ou un corps humain ou animal, tel que l'œil, la gencive, la peau, une plaie ou une brûlure ou un organe interne.
